(19) Europäisches Patentamt
European Patent Office
Office européen des brevets

(11) EP 0 847 751 B1

(12) FASCICULE DE BREVET EUROPEEN

(45) Date de publication et mention de la délivrance du brevet:
**03.04.2002 Bulletin 2002/14**

(51) Int Cl.[7]: **A61K 7/48**, A61K 7/42

(21) Numéro de dépôt: **97402612.2**

(22) Date de dépôt: **03.11.1997**

# (54) Composition cosmétique comprenant des composés minéraux photochromes

Kosmetische Zubereitungen, die photochrome minerale Verbindungen enthalten

Cosmetic compositions containing mineral photochromic compounds

(84) Etats contractants désignés:
**DE ES FR GB IT**

(30) Priorité: **16.12.1996 FR 9615451**

(43) Date de publication de la demande:
**17.06.1998 Bulletin 1998/25**

(73) Titulaire: **L'OREAL**
**75008 Paris (FR)**

(72) Inventeurs:
- **Remy, Christophe**
  **75020 Paris (FR)**
- **Mellul, Myriam**
  **94240 L'Hay Les Roses (FR)**

(74) Mandataire: **Dodin, Catherine**
**L'OREAL-DPI**
**6 rue Bertrand Sincholle**
**92585 Clichy Cédex (FR)**

(56) Documents cités:
**EP-A- 0 187 912**
**EP-A- 0 359 909**
**WO-A-92/10545**
**FR-A- 2 419 758**

- **DATABASE WPI Week 9549 Derwent Publications Ltd., London, GB; AN 95-380265 XP002039611 & JP 07 258 580 A (SHISEIDO CO LTD)**
- **DATABASE WPI Week 9542 Derwent Publications Ltd., London, GB; AN 95-325371 XP002039612 & JP 07 223 816 A (SHISEIDO CO LTD)**
- **DATABASE WPI Week 8828 Derwent Publications Ltd., London, GB; AN 88-194657 XP002039613 & JP 63 132 811 A (SHISEIDO CO LTD)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 847 751 B1

Printed by Jouve, 75001 PARIS (FR)

## Description

La présente invention se rapporte à une composition notamment cosmétique comprenant un composé minéral présentant des propriétés photochromes. Elle se rapporte également à l'utilisation dudit composé photochrome dans une composition cosmétique.

Les compositions cosmétiques, notamment les compositions de maquillage telles que les poudres libres ou compactes, les fonds de teint, les fards à joues ou à paupières, les rouges à lèvres ou les vernis à ongles, sont constituées d'un véhicule approprié et de différents agents de coloration destinés à conférer une certaine couleur auxdites compositions avant et/ou après leur application sur la peau, les muqueuses, les semi-muqueuses et/ou les phanères telles que les ongles ou les cheveux.
Pour créer des couleurs, on utilise aujourd'hui une gamme d'agents de coloration assez limitée et notamment des laques, des pigments minéraux ou des pigments nacrés. Les laques permettent d'obtenir des couleurs vives, mais pour la plupart sont instables à la lumière, à la température ou au pH. Certaines présentent également l'inconvénient de tacher la peau de manière disgracieuse après application, par dégorgement du colorant. Les pigments minéraux, en particulier les oxydes minéraux sont au contraire très stables mais donnent des couleurs plutôt ternes et pâles. Pour obtenir des effets colorés, on peut encore employer des pigments nacrés de couleurs variées, mais jamais intenses, qui permettent d'obtenir des effets irisés mais le plus souvent assez faibles.

Il a alors été proposé d'utiliser des composés photochromes dans les compositions de maquillage ou capillaire, de manière à obtenir des changements agréables et variables dans le 'rendu couleur' des maquillages de la peau et/ou des cheveux.
Les composés photochromes sont des composés qui possèdent la propriété de changer de couleur lorsqu'ils sont irradiés par une source lumineuse, puis de reprendre leur couleur initiale, ou une couleur proche, lorsque l'on arrête l'irradiation. De tels composés trouvent notamment une application particulièrement intéressante dans les compositions cosmétiques, en particulier dans les compositions de maquillage telles que les fonds de teint et les fards à joues ou à paupières. En effet, on a constaté que le 'rendu maquillage' d'une peau maquillée est différent selon que l'on se trouve en éclairage naturel ou en éclairage artificiel. Ainsi, un maquillage réalisé en éclairage artificiel paraîtra plus clair en lumière naturelle. A l'inverse, un maquillage réalisé à l'extérieur paraîtra plus sombre dans un endroit éclairé artificiellement.

Notamment, il a été proposé d'utiliser des composés photochromes organiques, tels que les composés de la famille des spiropyrannes ou des naphtooxazines.
Ces composés photochromes sont particulièrement intéressants dans la mesure où ils permettent d'obtenir un changement rapide de coloration du support sur lequel ils sont appliqués, lorsque ledit support est exposé aux UV par exemple, avec un retour rapide à la couleur initiale lorsque l'exposition aux UV cesse.
On peut ainsi citer le brevet FR1604929 qui décrit des compositions cosmétiques notamment capillaires sous forme d'aérosol, qui contiennent des composés photo-tropes tels que des nitrobenzylpyridines, des thiosemicarbazones ou des dérivés de spiropyrannes. Après sprayage de ces compositions sur les cheveux et exposition à la lumière du soleil, on obtient une coloration bleu-violet qui redevient jaune pâle dans l'obscurité.
Il a également été proposé, par exemple par le brevet EP359909, des compositions cosmétiques comprenant des composés photochromes minéraux particulier, choisis parmi les oxydes métalliques, leurs hydrates et leurs complexes. En particulier, ce document mentionne l'utilisation d'oxyde de titane traité de manière à le rendre photochrome, dans des compositions de maquillage telles que des poudres et des fonds de teint.

Toutefois, on a constaté que ces composés photochromes minéraux, bien qu'ils permettent d'obtenir un maquillage qui semble rester de couleur constante quel que soit l'éclairage, ne permettent toutefois pas d'obtenir un véritable changement de la couleur du maquillage, ou, en d'autres termes, un véritable changement du 'rendu maquillage'.
D'autre part, on a également constaté qu'après l'arrêt de l'irradiation lumineuse, la couleur du maquillage ne revenait pas toujours d'une manière acceptable à sa couleur initiale, en particulier qu'elle ne revenait pas complètement à une couleur identique à ladite couleur initiale.

L'invention a pour but de proposer une composition cosmétique comprenant un composé photochrome minéral particulier, ledit composé permettant l'obtention d'un changement de couleur rapide en fonction de la nature et/ou de l'intensité de la lumière, tout en conservant de bonnes propriétés cosmétiques à ladite composition.

La présente invention a donc pour objet une composition cosmétique comprenant au moins un composé photochrome minéral, caractérisée par le fait que ledit composé photochrome minéral est un aluminosilicate comprenant au moins un élément dopant.
L'invention a également pour objet l'utilisation d'un aluminosilicate comprenant au moins un élément dopant en tant que composé photochrome dans une composition cosmétique.

L'utilisation de composés photochromes selon l'invention permet d'obtenir un maquillage de couleur nette et pure, par rapport au maquillage obtenu avec des composés de l'art antérieur, étant donné que le signal lumineux, issu

du rayonnement incident lumineux sur lesdits composés, présente un spectre réémis plus étroit. Le 'rendu maquillage' obtenu parait donc plus intense.

Il est ainsi possible d'utiliser, dans la composition cosmétique, une quantité de composés photochromes plus faible que celle utilisée dans l'art antérieur, tout en obtenant un effet de maquillage et de couvrance comparable.

De plus, on a constaté que la nature chimique de l'élément dopant modifie les couleurs du composés photochrome. En conséquence, il est possible d'obtenir des compositions de maquillage présentant une gamme étendue de nuances, tout en utilisant des composés photochromes de la même famille, donc possédant les mêmes propriétés physico-chimiques, par exemple en terme de viscosité, rhéologie, taille des particules, etc. d'où une simplification de la mise en oeuvre des composés et de l'adaptation des formules.

Enfin, on a constaté que le changement de couleur obtenu à l'aide de ces composés était réversible, et permettait de revenir, après l'arrêt de l'irradiation par une source lumineuse, à la couleur initiale.

La composition selon l'invention comprend donc, en tant que composé photochrome, un aluminosilicate dopé.

L'aluminosilicate selon l'invention a une structure de base qui consiste en une cage formée par des tétraèdres de $AlO_4$ et de $SiO_4$, liés ensemble par leurs atomes d'oxygène.

Certains éléments chimiques peuvent être présents dans les cages ainsi formées. On appelle ces éléments, 'éléments dopants'.

L'aluminosilicate dopé présent dans la composition objet de la présente invention est donc un aluminosilicate qui comprend au moins un élément dopant.

Ces éléments dopants peuvent être des anions halogénures tels que les anions chlorure, iodure, bromure ou fluorure, seul ou en mélange.

Les éléments dopants peuvent également se présenter sous la forme des groupements soufre, sélénium, $SO_4^{2-}$, $WO_4^{2-}$ ou hydroxyle, ou encore sous la forme d'ions métalliques tels que les ions obtenus à partir du fer, chrome, manganèse, cobalt et/ou nickel.

On peut également utiliser un mélange de ces différents éléments dopants.

D'une manière générale, les composés photochromes susceptibles d'être utilisés dans la cadre de l'invention ont de préférence une structure de type

$$R_8Al_6Si_6O_{24}X_n$$

dans laquelle

- R représente un élément choisi parmi Na, K, Cs, Rb, Li, Ag ou Ca; de préférence Na, et
- X représente un ou plusieurs éléments dopants tels que définis ci-dessus, avec n pouvant être compris entre 1 et 5, de préférence compris entre 1 et 3.

On peut en particulier citer comme composés photochromes susceptibles d'être utilisés dans le cadre de la présente invention, ceux de la famille des sodalites qui ont pour formule

$$Na_8Al_6Si_6O_{24}X_2,$$

dans laquelle $X_2$ représente un ou plusieurs anions halogènes, et notamment $Cl_2$, ClBr, $I_2$ ou $Br_2$.

Certains de ces composés sont décrits dans l'article Williams et al, Journal of the American Ceramic Society, volume 52, pages 139-145 (mars 1969).

Ainsi, le dopage d'un tel composé, par exemple par des ions chlorures, pourrait permettre d'obtenir, par ajout à une composition initialement incolore, une composition susceptible de devenir, lors d'une irradiation aux rayons U. V., de couleur proche de la couleur magenta.

Lorsque le composé est dopé par des ions bromure ou iodure, par exemple, la couleur de la composition qui comprend le composé dopé peut tendre vers le pourpre ou le rouge.

L'utilisation d'un mélange de différents aluminosilicates, dopés par des anions de nature chimique variée, peut permettre d'obtenir une composition cosmétique qui présente des couleurs intermédiaires et variées. Ceci peut faciliter l'obtention de variations dans le rendu maquillage de la peau et/ou des cheveux.

L'aluminosilicate selon l'invention peut être incorporé dans la composition en une quantité déterminable aisément par l'homme du métier sur la base de ses connaissances générales, et qui peut notamment être comprise entre 0,01 et 30% en poids par rapport au poids total de la composition, de préférence en une quantité de 1 à 15% en poids.

La composition cosmétique selon l'invention peut se présenter sous la forme d'un produit à appliquer sur les muqueuses, les semi-muqueuses et/ou les tissus kératiniques tels que la peau et les phanères (ongles, cils, sourcils,

poils et cheveux).
En particulier, cette composition peut être un produit de soin et/ou de maquillage de la peau, un produit solaire ou autobronzant, voire un produit capillaire.
Les composés selon l'invention trouvent une application particulière dans le domaine des rouges à lèvres, des fonds de teint, des fards à joues ou à paupières, des eye-liners, des mascaras et des vernis à ongles aqueux ou solvant.

La composition selon l'invention contient en outre un milieu cosmétiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.
Ledit milieu peut comprendre ou se présenter sous la forme de, notamment, une suspension, une dispersion, une solution en milieu solvant ou hydroalcoolique, éventuellement épaissie voire gélifiée; une émulsion huile-dans-eau, eau-dans-huile, ou multiple; un gel ou une mousse; un gel émulsionné; une dispersion de vésicules notamment lipidiques; une lotion biphase ou multiphase; un spray; une poudre libre, compacte ou coulée; une pâte anhydre.

L'homme du métier pourra choisir la forme galénique appropriée, ainsi que sa méthode de préparation, sur base de ses connaissances générales, en tenant compte d'une part de la nature des constituants utilisés, notamment de leur solubilité dans le support, et d'autre part de l'application envisagée pour la composition. La composition cosmétique selon l'invention peut comprendre les constituants usuels au type d'application envisagé.

Ainsi, la composition selon l'invention peut comprendre une phase grasse, notamment constituée de corps gras liquides à 25°C, tels que des huiles d'origine animale, végétale, minérale ou synthétique; de corps gras solides à 25°C tels que des cires d'origine animale, végétale, minérale ou synthétique; de corps gras pâteux; de gommes; de leurs mélanges.

Les compositions selon l'invention peuvent ainsi comprendre des huiles volatiles, qui s'évaporeront au contact de la peau, mais dont la présence, dans la composition cosmétique, est utile car elles facilitent l'étalement de la composition lors de l'application sur la peau. De tels agents d'étalement appelés ici "huiles volatiles", sont généralement des huiles ayant, à 25°C, une tension de vapeur saturante au moins égale à 0,5 millibar (soit 50 Pa). De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.
On peut ainsi citer les huiles siliconées volatiles, telles que :

- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane, de l'hexylheptaméthyltrisiloxane ou de l'octylheptaméthyltrisiloxane.

On peut également citer les huiles volatiles hydrocarbonées, telles que les isoparaffines et notamment l'isododécane; et des huiles fluorées comme celle commercialisée sous la dénomination GALDEN® (MONTEFLUOS).

On peut également utiliser des huiles non volatiles, parmi lesquelles on peut citer:

- les polyalkyl($C_1$-$C_{20}$) siloxanes et notamment ceux à groupements terminaux triméthylsilyle, de préférence ceux dont la viscosité est inférieure à 0,06 $m^2$/s parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment celles de formule :

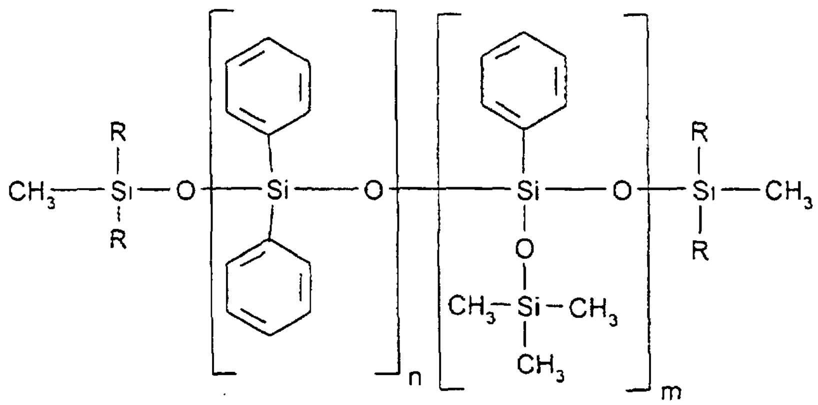

dans laquelle R est un radical alkyle en C1-C30, un radical aryle ou un radical aralkyle, n est un nombre entier compris entre 0 et 100, et m est un nombre entier compris entre 0 et 100, sous réserve que la somme est comprise entre 1 et 100.

- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule $R_1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance et/ou en texture. Ces corps gras additionnels peuvent être des cires, des gommes et/ou des corps gras pâteux d'origine animale, végétale, minérale ou synthétique, ainsi que leurs mélanges.

On peut notamment citer :

- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

La composition selon l'invention peut également comprendre un ou plusieurs solvants organiques cosmétiquement acceptables (tolérance, toxicologie et toucher acceptables). Ces solvants organiques peuvent représenter de 0 % à 98 % du poids total de la composition. Ils peuvent être choisis dans le groupe constitué par les solvants organiques hydrophiles, les solvants organiques lipophiles, les solvants amphiphiles ou leurs mélanges.
Parmi les solvants organiques hydrophiles, on peut citer par exemple des monoalcools inférieurs linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di- alkyle d'isosorbide dont les groupements alkyle ont de 1 à 5 atomes de carbone ; les éthers de glycol comme le diéthylène glycol mono-méthyl ou mono-éthyl éther et les éthers de propylène glycol comme le dipropylène glycol méthyl éther.
Comme solvants organiques amphiphiles, on peut citer des polyols tels des dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle, le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl-décyle, le palmitate de 2-octyl-décyle, le myristate de 2-octyl-dodécyle, le succinate de di-(éthyl-2-hexyle), le malate de diisostéaryle, le lactate de 2-octyl-dodécyle, le triisostéarate de glycérine, le triisostéarate de diglycérine.

La composition selon l'invention peut également comprendre une phase aqueuse, qui peut comprendre de l'eau, une eau florale telle que l'eau de bleuet et/ou une eau minérale telle que l'eau de VITTEL, l'eau de LUCAS ou l'eau de LA ROCHE POSAY.

Ladite phase aqueuse peut comprendre de 0 % à 14 % en poids, par rapport au poids total de la phase aqueuse, d'un monoalcool inférieur en $C_2$-$C_6$ et/ou d'un polyol tel que le glycérol, le butylèneglycol, l'isoprène glycol, le propylèneglycol, le polyéthylèneglycol.

Lorsque la composition selon l'invention se présente sous la forme d'une émulsion, elle peut éventuellement comprendre en outre un tensioactif, de préférence en une quantité de 0,01 à 30% en poids par rapport au poids total de la composition.

Parmi les tensioactifs anioniques qui peuvent être utilisés seuls ou en mélange, on peut citer en particulier les sels alcalins, les sels d'ammonium, les sels d'amines ou les sels d'aminoalcools des composés suivants : les alcoylsulfates, alcoyléther sulfates, alcoylamides sulfates et éthers sulfates, alcoylarylpolyéthersulfates, monoglycérides sulfates, alcoylsulfonates, alcoylamides sulfonates, alcoylarylsulfonates, α-oléfines sulfonates, paraffines sulfonates, alcoylsulfosuccinates, alcoyléthersulfosuccinates, alcoylamides sulfosuccinates, alcoylsulfosuccinamates, alcoylsulfoacétates, alcoylpolyglycérol carboxylates, alcoylphosphates/alcoylétherphosphates, acylsarcosinates, alcoylpolypeptidates, alcoylamidopolypeptidates, acyliséthionates, alcoyllaurates.

Le radical alcoyle ou acyle dans tous ces composés désigne généralement une chaîne de 12 à 18 atomes de carbone.

On peut aussi citer les savons et les sels d'acides gras tels que les acides oléique, ricinoléique, palmitique, stéarique, les acides d'huile de coprah ou d'huile de coprah hydrogénée et notamment les sels d'amines tels que les stéarates d'amines; les acyl lactylates dont le radical acyle comprend 8-20 atomes de carbone; les acides carboxyliques d'éthers polyglycoliques répondant à la formule :

> $Alk\text{-}(OCH_2\text{-}CH_2)_n\text{-}OCH_2\text{-}COOH$ sous forme acide ou salifiée où le substituant Alk correspond à une chaîne linéaire ayant de 12 à 18 atomes de carbone et où n est un nombre entier compris entre 5 et 15.

Parmi les tensioactifs non ioniques qui peuvent être utilisés seuls ou en mélange, on peut citer en particulier : les alcools, les alcoylphénols et acides gras polyéthoxylés, polypropoxylés ou polyglycérolés à chaîne grasse comportant 8 à 18 atomes de carbone; des copolymères d'oxydes d'éthylène et de propylène, des condensats d'oxyde d'éthylène et de propylène sur des alcools gras, des amides gras polyéthoxylés, des amines grasses polyéthoxylées, des éthanolamides, des esters d'acides gras de glycol, des esters d'acides gras du sorbitan oxyéthylénés ou non, des esters d'acides gras du saccharose, des esters d'acides gras de polyéthylèneglycol, des triesters phosphoriques, des esters d'acides gras de dérivés de glucose; les alkylpolyglycosides et les alkylamides des sucres aminés; les produits de condensation d'un α-diol, d'un monoalcool, d'un alcoylphénol, d'un amide ou d'un diglycolamide avec le glycidol ou un précurseur de glycidol.

La composition selon l'invention peut également comprendre 0 à 5 % en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut encore comprendre un ou plusieurs agents épaississants dans des concentrations préférentielles allant de 0 à 6 % en poids, par rapport au poids total de l'émulsion. L'agent épaississant peut être choisi parmi :

- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxyméthylcellulose, les dérivés de l'amidon, les dérivés d'éthers de cellulose possédant des groupes ammonium quaternaires, les polysaccharides cationiques;
- les polymères synthétiques comme les acides polyacryliques tels que les polymères poly(méth)acrylates de glycéryl tels que l'HISPAGEL ou le LUBRAGEL des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C de HOECHST; les copolymères acrylate/octylacrylamide tels que le Dermacryl de National Starch; les polymères à base de polyacrylamide tels que le SEPIGEL 305 de SEPPIC, les polymères réticulés d'acrylamide et de chlorure de méthacryloyloxyéthyltriméthylammonium tels que le SALCARE SC 92 de ALLIED COLLOIDS,
- le silicate de magnésium et d'aluminium.

L'agent épaississant peut être présent dans la phase grasse et/ou dans la phase aqueuse.

Selon l'application envisagée, la composition peut comprendre en outre un polymère filmogène. Ceci est notamment le cas lorsque l'on souhaite préparer une composition de type vernis à ongles, mascara, eye-liner ou composition capillaire de type laque.

Les polymères peuvent être dissous ou dispersés dans le milieu cosmétiquement acceptable. En particulier, le polymère peut être présent sous forme de solution dans un solvant organique ou sous forme de dispersion aqueuse de particules de polymère filmogène.

Ledit polymère peut être choisi parmi la nitrocellulose, l'acétobutyrate de cellulose, les butyralpolyvinyliques, les résines alkydes, les polyesters, les acryliques, les vinyliques, et/ou les polyuréthannes.

On peut en particulier citer les copolymères d'acide (méth)acrylique et d'au moins un monomère ester d'acide (méth) acrylique linéaire, ramifié ou cyclique et/ou d'au moins un monomère amide d'acide (méth)acrylique mono ou di-substitué linéaire, ramifié ou cyclique; les copolymères acide (méth)acrylique/(meth)acrylate de tertio-butyle et/ou (méth) acrylate d'isobutyle/(méth)acrylate d'alkyle en $C_1$-$C_4$; les terpolymères et tétrapolymères acide (meth)acrylique/acrylate d'éthyle/ méthacrylate de méthyle; les tétrapolymères méthacrylate de méthyle/acrylate de butyle ou d'éthyle/acrylate ou méthacrylate d'hydroxyéthyle ou de 2-hydroxypropyle/acide (meth)acrylique; les copolymères d'acide acrylique et de méthacrylate d'alkyle en $C_1$-$C_4$ ; les terpolymères de vinyl pyrrolidone, d'acide acrylique et de méthacrylate d'alkyle en $C_{1-20\,;}$ les copolymères amphotères; les esters vinyliques d'acide ramifiés; les esters vinyliques d'acide benzoïque; les copolymères d'acide (meth)acrylique et d'au moins un monomère oléfinique; les copolymères de monoacide vinylique et/ou de monoacide allylique.

Parmi les résines, on peut citer les résines du type aryl-sulfonamide formaldéhyde ou aryl-sulfonamide époxy; les résines du type acrylique, styrénique, acrylate-styrénique et vinylique.

La composition peut également comprendre au moins un plastifiant, tel que le phosphate de tricrésyle, le benzoate de benzyle, le citrate de triéthyle, le citrate de tributyle, l'acétylcitrate de triéthyle, l'acétylcitrate de triétyl-2 hexyle, le camphre; les éthers de glycol; l'huile de ricin oxyéthylénée à 40 moles d'oxyde d'éthylène; le propylène glycol; le butyl glycol ; l'éthylène glycol monométhyléther acétate; les éthers de propylène glycol; les éthers esters de propylène glycol et d'éthylène glycol; les esters de diacides tels que les phtalates et adipates de diéthyle, dibutyle et de diisopropyle, les tartrates de diéthyle et dibutyle, les succinates de diéthyle et de dibutyle, les sébacates de diéthyle et de dibutyle, les phosphates de diéthyle, de dibutyle et de diéthyl-2 hexyle, l'acétyl citrate de diéthyle ou de dibutyle; les esters de glycérol. Les plastifiants peuvent être généralement présents à une teneur allant de 1 % à 40 % en poids par rapport au poids total de la composition.

Lorsque les compositions se présentent sous forme d'un vernis à ongles anhydre, le système solvant peut représenter environ de 55 % à 90 % en poids par rapport au poids total du vernis. Ce système solvant peut être constitué par un mélange de divers solvants organiques volatils tels que les cétones notamment l'acétone, la méthyléthylcétone, la méthylisobutylcétone; les acétates notamment l'acétate d'éthyle, l'acétate de butyle, l'acétate de méthoxy-2 éthyle, l'acétate de méthyle, l'acétate d'amyle et l'acétate d'isopropyle. Le système solvant peut également comprendre un diluant tel que l'hexane ou l'octane ou encore un hydrocarbure aromatique tel que le toluène ou le xylène en une proportion notamment de 10 à 35 % en poids par rapport au poids total du vernis.

La composition peut comprendre en outre une phase particulaire, qui peut comprendre des pigments et/ou des nacres et/ou des charges habituellement utilisés dans les compositions cosmétiques.

Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Par nacres, il faut comprendre des particules irisées qui réfléchissent la lumière.

Les pigments peuvent être présents dans la composition à raison de 0 à 15% en poids de la composition finale, et de préférence à raison de 8 à 10% en poids. Ils peuvent être blancs ou colorés, minéraux et/ou organiques, de taille usuelle ou nanométrique. Ils peuvent se présenter sous forme de poudre ou de pâte pigmentaire.

On peut citer les dioxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium, le noir de carbone. On peut encore citer les laques couramment employées pour conférer aux lèvres et à la peau un effet de maquillage, qui sont des sels de calcium, de baryum, d'aluminium ou de zirconium, de colorants acides tels que les colorants halogéno-acides, azoïques, anthraquinoniques, etc.

Les nacres peuvent être présentes dans la composition à raison de 0 à 20% en poids, de préférence à un taux de l'ordre de 8 à 15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Les charges, qui peuvent être présentes à raison de 0 à 30% en poids, de préférence 5 à 15%, dans la composition, peuvent être minérales ou de synthèse, lamellaires ou non lamellaires. On peut citer le talc, le mica, la silice, le kaolin,

les poudres de Nylon et de polyéthylène, le Téflon, l'amidon, le nitrure de bore, les microsphères de polymère telles que l'Expancel (Nobel Industrie), le polytrap (Dow Corning) et les microbilles de résine de silicone (Tospearls de Toshiba, par exemple), le carbonate de calcium précipité, le carbonate ou l'hydrocarbonate de magnésium, des savons métalliques dérivés d'acides organiques carboxyliques ayant de 8 à 22 atomes de carbone.
exemple).

Selon le type de formulation, la phase pulvérulente peut représenter de 0,01 à 99% en poids de la composition.

La composition peut en outre comprendre un colorant, notamment un colorant organique naturel tel que le carmin de cochenille, et/ou un colorant de synthèse tel que les colorants halogéno-acides, azoïques, anthraquinoniques. On peut également citer des colorants minéraux tels que le sulfate de cuivre.

La composition peut comprendre en outre tout additif usuellement utilisé dans le domaine cosmétique, tel que des antioxydants, des parfums, des huiles essentielles, des conservateurs, des actifs cosmétiques lipophiles ou hydrophiles, des hydratants, des vitamines, des colorants, des acides gras essentiels, des sphingolipides, des agents autobronzants tels que la DHA, des filtres solaires, des agents antimousses, des agents séquestrants, des antioxydants.
Bien entendu l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.

Les compositions cosmétiques selon l'invention sont essentiellement celles concernant le maquillage du visage, c'est-à-dire les fards à paupières ou à joues, les eye-liners, les mascaras, les poudres, les fonds de teint, les crèmes teintées, les rouges à lèvres, mais également le maquillage des cheveux notamment des gels, crèmes ou mousses pour la coloration temporaire des cheveux, et le maquillage des ongles notamment les vernis à ongles anhydres ou aqueux.

L'invention est illustrée plus en détail dans les exemples suivants.

Exemple 1 : Fond de teint huile-dans-eau

| Phase A : | |
|---|---|
| Acide stéarique | 2% |
| Stéarate de glycéryle | 3% |
| Isostéarate de glycéryle | 2% |
| Huile minérale | 8% |
| Diméthicone | 4% |
| Phase B : | |
| Triéthanolamine | 1% |
| Phase C : | |
| Composé photochrome $Na_8Al_6Si_6O_{24}Cl_2$ | 10% |
| Gomme de cellulose | 3,5% |
| Lauroyl sarcosinate de sodium | 3,5% |
| Glycérine | 2% |
| Eau | qsp 100% |

On prépare les phases A et C séparément, on les chauffe à 80°C puis on les homogénéise au Moritz. On verse B sur le mélange sous agitation et on poursuit l'agitation jusqu'à complet refroidissement.
On obtient un fond de teint ayant de bonnes propriétés cosmétiques.

Exemple 2 : Mascara

| Phase A : | |
|---|---|
| acide stéarique | 6% |

(suite)

| | |
|---|---|
| Phase A : | |
| stéarate de glycéryle<br>mélange de cires | 4%<br>16% |
| Phase B: | |
| composé photochrome $Na_8Al_6Si_6O_{24}I_2$<br>ultramarines et silice | 4%<br>1% |
| Phase C : | |
| hydroxyéthylcellulose<br>eau | 0,2%<br>qsp 100% |
| Phase D: | |
| triéthanolamine | 3% |
| Phase E: | |
| acacia | 5,8% |

On prépare C sous agitation, à chaud (environ 80°C) et on laisse reposer pendant 12 heures. On prépare la phase E en évitant de chauffer. On chauffe A à 120°C puis on l'homogénéise par passage au Moritz. On ajoute B dans A et on poursuit l'agitation pendant 10mn. On chauffe C+D et E à 80°C et on émulsionne avec A+B. On laisse homogénéiser au Moritz jusqu'à ce que le mélange prenne en masse. On refroidit sous agitation lente.
On obtient un mascara de couleur agréable, ayant de bonnes propriétés cosmétiques.

Exemple 3 : Composition de vernis à ongles

| | |
|---|---|
| Nitrocellulose | 10,8% |
| Résine toluène sulfonamide formaldéhyde | 10,7% |
| Acétyl citrate de tributyle | 6,5% |
| Toluène | 30% |
| Acétate de butyle | 20% |
| Acétate d'éthyle | 10% |
| Alcool isopropylique | 5% |
| Stéarakonium hectorite | 1,3% |
| Composé photochrome $Na_8Al_6Si_6O_{24}Br_2$ | 1% |
| Acide citrique | 0,1% |

On obtient un vernis de couleur agréable, ayant de bonnes propriétés cosmétiques.

Exemple 4 : Composition de rouge à lèvres

| | |
|---|---|
| Esters d'alcools gras en C8-C10 | 30% |
| Alcools gras en C10 | 7% |
| Amidon octylsuccinate d'aluminium | 10% |
| Ozokérite | 10% |
| Cire de Carnauba | 4% |
| Cire d'abeille | 3% |
| Dioxyde de titane | 3% |
| Composé photochrome | 1% |

(suite)

| | |
|---|---|
| Huile de ricin | qsp 100 % |

On obtient un rouge à lèvres de couleur agréable, ayant de bonnes propriétés cosmétiques.

Exemple 5 : Composition de fard à paupières

On prépare une composition de fard à paupière à partir des formules données ci-dessous

| | |
|---|---|
| Talc | qsp 100 % |
| Mica | 22% |
| Oxychlorure de bismuth | 8% |
| Stéarate de zinc | 3% |
| Nylon-12 | 20% |
| Composé photochrome | 4% |
| Huile de vaseline | 3% |
| Myristate d'isopropyle | 3% |
| $TiO_2$ | 8% |

Préparation du fard à paupières : On broie les charges avec les pigments, on ajoute ie liant (huile de vaseline et myristate d'isopropyle) et on broie à nouveau. Les formules sont compactées dans des coupelles métalliques.
On obtient un fard à paupières de couleur agréable, ayant de bonnes propriétés cosmétiques.

## Revendications

1. Composition cosmétique comprenant au moins un composé photochrome minéral, **caractérisée par le fait que** ledit composé photochrome minéral est un aluminosilicate comprenant au moins un élément dopant.

2. Composition selon la revendication 1, dans laquelle l'élément dopant est choisi parmi les anions halogénures tels que les anions chlorure, iodure, bromure ou fluorure, seul ou en mélange; les groupements soufre, sélénium, $SO_4^{2-}$, $WO_4^{2-}$ ou hydroxyle; les ions métalliques, notamment obtenus à partir du fer, chrome, manganèse, cobalt et/ou nickel; leurs mélanges.

3. Composition selon l'une des revendications précédentes, dans laquelle le composé photochrome a une structure de type

$$R_8Al_6Si_6O_{24}X_n$$

dans laquelle

- R représente un élément choisi parmi Na, K, Cs, Rb, Li, Ag ou Ca; de préférence Na, et
- X représente un ou plusieurs éléments dopants tels que définis ci-dessus, avec n pouvant être compris entre 1 et 5, de préférence compris entre 1 et 3.

4. Composition selon l'une des revendications précédentes, dans laquelle le composé photochrome a pour formule

$$Na_8Al_6Si_6O_{24}X_2$$

dans laquelle $X_2$ représente un ou plusieurs anions halogènes, et notamment $Cl_2$, ClBr, $I_2$ ou $Br_2$.

5. Composition selon l'une des revendications précédentes, dans laquelle le composé photochrome est présent en une quantité comprise entre 0,01 et 30% en poids par rapport au poids total de la composition, de préférence en

une quantité de 1 à 15% en poids.

**6.** Composition selon l'une des revendications précédentes, comprenant en outre au moins un composé choisi parmi les huiles éventuellement volatiles, d'origine animale, végétale, minérale ou synthétique; les cires d'origine animale, végétale, minérale ou synthétique; les corps gras pâteux; les gommes; les solvants organiques cosmétiquement acceptables; l'eau; les tensioactifs; les co-émulsionnants; les épaississants; les polymères filmogènes; les pigments; les nacres; les charges; les colorants.

**7.** Composition selon l'une des revendications précédentes, se présentant sous la forme d'un produit à appliquer sur les muqueuses, les semi-muqueuses et/ou les tissus kératiniques tels que la peau et les phanères.

**8.** Composition selon l'une des revendications précédentes, se présentant sous la forme d'un produit de soin et/ou de maquillage de la peau, d'un produit solaire, d'un autobronzant, d'un produit capillaire.

**9.** Composition selon l'une des revendications précédentes, se présentant sous la forme d'un rouge à lèvres, d'un fond de teint, d'un fard à joues ou à paupières, d'un eye-liner, d'un mascara, d'un vernis à ongles aqueux ou solvant.

**10.** Utilisation d'un aluminosilicate comprenant au moins un élément dopant en tant que composé photochrome dans une composition cosmétique.

**11.** Utilisation selon la revendication 10, dans laquelle l'élément dopant est choisi parmi les anions halogénures tels que les anions chlorure, iodure, bromure ou fluorure, seul ou en mélange; les groupements soufre, sélénium, $SO_4^-$, $WO_4^-$ ou hydroxyle; les ions métalliques, notamment obtenus à partir du fer, chrome, manganèse, cobalt et/ou nickel; leurs mélanges.

**12.** Utilisation selon l'une des revendications 10 à 11, dans laquelle le composé photochrome a une structure de type

$$R_8Al_6Si_6O_{24}X_n$$

dans laquelle

- R représente un élément choisi parmi Na, K, Cs, Rb, Li, Ag ou Ca; de préférence Na, et
- X représente un ou plusieurs éléments dopants tels que définis ci-dessus, avec n pouvant être compris entre 1 et 5, de préférence compris entre 1 et 3.

**13.** Utilisation selon l'une des revendications 10 à 12, dans laquelle le composé photochrome a pour formule

$$Na_8Al_6Si_6O_{24}X_2$$

dans laquelle $X_2$ représente un ou plusieurs anions halogènes, et notamment $Cl_2$, ClBr, $I_2$ ou $Br_2$.

**14.** Utilisation selon l'une des revendications 10 à 13, dans laquelle le composé photochrome est présent à raison de 0,01-30% en poids par rapport au poids total de la composition, de préférence en une quantité de 1 à 15% en poids.

**15.** Utilisation selon l'une des revendications 10 à 14, dans une composition se présentant sous la forme d'un produit de soin et/ou de maquillage de la peau, d'un produit solaire, d'un autobronzant, d'un produit capillaire.

**Patentansprüche**

**1.** Kosmetische Zusammensetzung, die mindestens eine anorganische photochrome Verbindung enthält, **dadurch gekennzeichnet, daß** die anorganische photochrome Verbindung ein Aluminosilicat ist, das mindestens einen Dotierstoff enthält.

**2.** Zusammensetzung nach Anspruch 1, wobei der Dotierstoffunter den Halogenidanionen, beispielsweise den Anionen Chlorid, Iodid, Bromid oder Fluorid einzeln oder im Gemisch; den Gruppen Schwefel, Selen, $SO_4^{2-}$, $WO_4^{2-}$

oder Hydroxy; den Metallionen und insbesondere den ausgehend von Eisen, Chrom, Mangan, Cobalt und/oder Nickel erhaltenen Ionen; und deren Gemischen ausgewählt ist.

**3.** Zusammensetzung nach einem der vorhergehenden Ansprüche, wobei die photochrome Verbindung eine Struktur des folgenden Typs hat:

$$R_8Al_6Si_6O_{24}X_n$$

worin bedeuten

- R eine Element, das unter Na, K, Cs, Rb, Li, Ag oder Ca und vorzugsweise Na ausgewählt ist, und
- X einen oder mehrere der oben definierten Dotierstoffe, wobei n im Bereich von 1 bis 5 und vorzugsweise 1 bis 3 liegen kann.

**4.** Zusammensetzung nach einem der vorhergehenden Ansprüche, wobei die photochrome Verbindung die folgende Formel hat:

$$Na_8Al_6Si_6O_{24}X_2,$$

worin $X_2$ ein oder mehrere Halogenanionen und insbesondere $Cl_2$, ClBr, $I_2$ oder $Br_2$ bedeutet.

**5.** Zusammensetzung nach einem der vorhergehenden Ansprüche, wobei die photochrome Verbindung in einer Menge von 0,01 bis 30 Gew.-%, bezogen auf das Gesamtgewicht der Zusammensetzung, und vorzugsweise in einer Menge von 1 bis 15 Gew.-% vorliegt.

**6.** Zusammensetzung nach einem der vorhergehenden Ansprüche, wobei sie ferner mindestens eine Verbindung enthält, die unter den gegebenenfalls flüchtigen Ölen tierischer, pflanzlicher, mineralischer oder synthetischer Herkunft; Wachsen tierischer, pflanzlicher, mineralischer oder synthetischer Herkunft; pastösen Fettsubstanzen; Gummen; organischen, kosmetisch akzeptablen Lösungsmitteln; Wasser; grenzflächenaktiven Stoffen; Coemulgatoren; Verdickungsmitteln; filmbildenden Polymeren; Pigmenten; Perlglanzpigmenten; Füllstoffen; und Farbmitteln ausgewählt ist.

**7.** Zusammensetzung nach einem der vorhergehenden Ansprüche, die in Form eines Produktes vorliegt, das auf die Schleimhäute, die Semischleimhäute und/oder die Keratinsubstanzen, wie die Haut und die Hautanhangsgebilde, aufgetragen werden soll.

**8.** Zusammensetzung nach einem der vorhergehenden Ansprüche, die als Produkt zur Pflege und/oder zum Schminken der Haut, Sonnenschutzmittel, Selbstbräunungsmittel oder Produkt für das Haar vorliegt.

**9.** Zusammensetzung nach einem der vorhergehenden Ansprüche, die als Lippenstift, Make-up, Wangenrouge, Lidschatten, Eyeliner, Mascara oder wäßriger oder lösungsmittelhaltiger Nagellack vorliegt.

**10.** Verwendung eines Aluminosilicats, das mindestens einen Dotierstoff enthält, als photochrome Verbindung in einer kosmetischen Zusammensetzung.

**11.** Verwendung nach Anspruch 10, wobei der Dotierstoff unter den Halogenidanionen, beispielsweise den Anionen Chlorid, Iodid, Bromid oder Fluorid einzeln oder im Gemisch; den Gruppen Schwefel, Selen, $SO_4^{2-}$, $WO_4^{2}$ oder Hydroxy; Metallionen und insbesondere ausgehend von Eisen, Chrom, Mangan, Cobalt und/oder Nickel erhaltenen Ionen; und deren Gemischen ausgewählt ist.

**12.** Verwendung nach einem der Ansprüche 10 bis 11, wobei die photochrome Verbindung eine Struktur des folgenden Typs aufweist:

$$R_8Al_6Si_6O_{24}X_n$$

worin bedeuten:

- R eine Element, das unter Na, K, Cs, Rb, Li, Ag oder Ca und vorzugsweise Na ausgewählt ist, und
- X einen oder mehrere der oben definierten Dotierstoffe, wobei n im Bereich von 1 bis 5 und vorzugsweise 1 bis 3 liegen kann.

**13.** Verwendung nach einem der Ansprüche 10 bis 12, wobei die photochrome Verbindung die folgende Formel hat:

$$Na_8Al_6Si_6O_{24}X_2,$$

worin $X_2$ ein oder mehrere Halogenanionen und insbesondere $Cl_2$, ClBr, $I_2$ oder $Br_2$ bedeutet.

**14.** Verwendung nach einem der Ansprüche 10 bis 13, wobei die photochrome Verbindung in einer Menge von 0,01 bis 30 Gew.-%, bezogen auf das Gesamtgewicht der Zusammensetzung, und vorzugsweise in einer Menge von 1 bis 15 Gew.-% vorliegt.

**15.** Verwendung nach einem der Ansprüche 10 bis 14, in einer Zusammensetzung, die als Produkt zur Pflege und/ oder zum Schminken der Haut, Sonnenschutzmittel, Selbstbräunungsmittel oder Produkt für das Haar vorliegt.

**Claims**

**1.** Cosmetic composition comprising at least one inorganic photochromic compound, **characterized in that** the said inorganic photochromic compound is an aluminosilicate comprising at least one doping element.

**2.** Composition according to Claim 1, in which the doping element is chosen from halide anions such as chloride, iodide, bromide or fluoride anions, alone or as a mixture; sulphur, selenium, $SO_4^{2-}$, $WO_4^{2-}$ or hydroxyl groups; metal ions obtained in particular from iron, chromium, manganese, cobalt, and/or nickel; mixtures thereof.

**3.** Composition according to either of the preceding claims, in which the photochromic compound has a structure of the type

$$R_8Al_6Si_6O_{24}X_n$$

in which

- R represents an element chosen from Na, K, Cs, Rb, Li, Ag or Ca; preferably Na, and
- X represents one or more doping elements as defined above, with it being possible for n to be between 1 and 5, preferably between 1 and 3.

**4.** Composition according to one of the preceding claims, in which the photochromic compound has the formula

$$Na_8Al_6Si_6O_{24}X_2$$

in which $X_2$ represents one or more halogen anions, and in particular $Cl_2$, ClBr, $I_2$ or $Br_2$.

**5.** Composition according to one of the preceding claims, in which the photochromic compound is present in an amount of between 0.01 and 30% by weight relative to the total weight of the composition, preferably in an amount of from 1 to 15% by weight.

**6.** Composition according to one of the preceding claims, also comprising at least one compound chosen from oils which may be volatile, of animal, plant, mineral or synthetic origin; waxes of animal, plant, mineral or synthetic origin; pasty fatty substances; gums; cosmetically acceptable organic solvents; water; surfactants; co-emulsifiers; thickeners; film-forming polymers; pigments; pearlescent agents; fillers; dyes.

**7.** Composition according to one of the preceding claims, in the form of a product to be applied to mucous membranes, semi-mucous membranes and/or keratin tissues such as the skin and the exoskeleton.

**8.** Composition according to one of the preceding claims, in the form of a care and/or make-up product for the skin, an antisun product, a self-tanning product or a hair product.

**9.** Composition according to one of the preceding claims, in the form of a lipstick, a foundation, a blusher, an eyeshadow, an eye-liner, a mascara or an aqueous or solvent-based nail varnish.

**10.** Use of an aluminosilicate comprising at least one doping element, as photochromic compound in a cosmetic composition.

**11.** Use according to Claim 10, in which the doping element is chosen from halide anions such as chloride, iodide, bromide or fluoride anions, alone or as a mixture; sulphur, selenium, $SO_4^{2-}$, $WO_4^{2-}$ or hydroxyl groups; metal ions obtained in particular from iron, chromium, manganese, cobalt, and/or nickel; mixtures thereof.

**12.** Use according to either of Claims 10 and 11, in which the photochromic compound has a structure of the type

$$R_8Al_6Si_6O_{24}X_n$$

in which

- R represents an element chosen from Na, K, Cs, Rb, Li, Ag or Ca; preferably Na, and
- X represents one or more doping elements as defined above, with it being possible for n to be between 1 and 5, preferably between 1 and 3.

**13.** Use according to one of Claims 10 to 12, in which the photochromic compound has the formula

$$Na_8Al_6Si_6O_{24}X_2$$

in which $X_2$ represents one or more halogen anions, and in particular $Cl_2$, ClBr, $I_2$ or $Br_2$.

**14.** Use according to one of Claims 10 to 13, in which the photochromic compound is present in an amount of between 0.01 and 30% by weight relative to the total weight of the composition, preferably in an amount of from 1 to 15% by weight.

**15.** Use according to one of Claims 10 to 14, in a composition in the form of a care and/or make-up product for the skin, an antisun product, a self-tanning product or a hair product.